# EUROPEAN PATENT APPLICATION

(11) **EP 4 589 226 A1**
(43) Date of publication of application: **23.07.2025**
(21) Application number: 24152832.2
(22) Date of filing: 19.01.2024
(51) Int. Cl.: F25D 19/00, A61N 5/00, F17C 3/08, H05H 7/00, H01F 6/04, H05H 13/00

(54) **DEVICE FOR COOLING AN OBJECT IN A VACUUM CHAMBER**

(71) Applicant: Ion Beam Applications, 1348 Louvain-la-Neuve (BE)
(72) Inventor: NUTTENS, Vincent, 1348 Louvain-la-Neuve (BE); GUILLAUME, Colin, 1348 Louvain-la-Neuve (BE); COLLIGNON, Guillaume, 1348 Louvain-la-Neuve (BE)
(74) Representative: Icosa Europe

(57) **Abstract**

The present disclosure relates to a device (1) for cooling an object (100) contained into a vacuum chamber (200), the object being a superconducting magnet for example. The cooling device is insertable into and removable out of a boot (300) housed by the vacuum chamber (200), the boot (300) or part of it being in thermal contact with the object to be cooled (100). A distal portion (4) of the cooling device comprises a first cold station (10) and a first coupler (20) thermally connected to the first cold station. The first coupler (20) comprises at least two mobile contacts (22, 60) that are thermally connected to the first cold station (10), and the cooling device comprises a driving means (50) and a mechanical transmission connecting the driving means to the at least two mobile contacts (22, 60), the driving means and the mechanical transmission being configured to move each of the at least two mobile contacts (22, 60) radially inwardly and outwardly in order to make or loosen a conductive thermal contact between the first cold station (10) and the boot (300) or part thereof.

## Description

### FIELD OF INVENTION

The present disclosure relates to a device for cooling an object contained in a vacuum chamber, such as for cooling a superconducting magnet enclosed in a vacuum chamber.

### BACKGROUND OF INVENTION

Devices for cooling an object enclosed in a vacuum chamber, such as for cooling a superconducting magnet for example, are generally known in the art.

In the field of charged particle therapy for example, systems are known for generating and transporting a charged particle beam from a particle accelerator to a patient to be treated with said particle beam. A particle accelerator for use in a charged particle therapy system, such as a cyclotron or a synchrocyclotron for example, may comprise a superconducting magnet to generate the main magnetic field in the accelerator. A superconducting magnet is an electromagnet whose coils are made of superconducting wire that must be cooled to cryogenic temperatures during operation. In a particle accelerator, the main superconducting magnet is housed in a thermally insulated container held under vacuum, sometimes called a cryostat. Optionally, the main superconducting magnet may be surrounded by a heat shield which serves to intercept heat radiated by the container before it reaches the superconducting magnet. The main superconducting magnet (or its coils) is thermally linked to a cryogenic refrigerator, sometimes called a cryocooler.

Such cryostats and cryocoolers are well known in the art. The cryocooler is generally an independent device which can be inserted into a separate chamber or boot of the cryostat and removed from the chamber or boot of the cryostat for maintenance and/or repair.

US 8,291,717 discloses a cryocooler to cool a superconducting magnet contained in a cryostat and that allows for replacement or repair without the need to break the cryostat vacuum or the need to warm up the superconducting magnet. After insertion of the cryocooler into a boot of the cryostat, a driving means establishes a thermo-mechanical coupling between a cold station of the cryocooler and a conductive thermal link to the superconducting magnet, via a wall of the boot, by pushing the cold station of the cryocooler cross-axially to make contact with the inner wall of the boot or with a part thereof.
The maintenance and/or repair of such a system is however not convenient, particularly since the driving means can't be easily removed or accessed, as it is connected to the boot. The centering of the cryocooler into the boot after its insertion into the boot is also not easy to achieve and may require the use of expensive bellows that significantly increase the complexity of the cryocooler support structure. The disengagement and retraction of the cryocooler from the boot of the cryostat may also be problematic, particularly if the thermo-mechanical coupling between the cold station of the cryocooler and the boot becomes stuck due to frozen condensation.

US10495261 discloses another example of a removable cryocooler to cool a superconducting magnet contained in a cryostat. In this device, the cryocooler has a clamping ring made of PTFE, which, due to its radial contraction when it is cooled down, pushes a cup-shaped portion of a cold station of the cryocooler against a cylindrical terminal of a conductive thermal link to the superconducting magnet. The clamping ring is surrounded by a heating wire to heat up the clamping ring so that it may expand radially in order to release the pressure on the cup-shaped portion and hopefully to release the thermal contact between the cup-shaped portion of the cryocooler and the cylindrical terminal of the conductive thermal link.
The cooling down and/or the heating up of the clamping ring may however take some time before it has sufficiently increased or released its pressure on the cup-shaped portion and hence before the cryocooler can effectively be used or removed from the boot. It may also be problematic to know when the thermal contact is sufficiently established or when it is sufficiently released. The disengagement and retraction of the cryocooler from the boot of the cryostat may also be problematic, particularly if the coupling between the cup-shaped portion of the cryocooler and the cylindrical terminal of the thermal link becomes stuck due to frozen condensation. Moreover, in case of failure of the heating system of the cryocooler, the thermal contact can no longer be released and the cryocooler can no longer be disconnected from the boot without warming up the whole system, which takes a lot of time during which it can't be used operationally.

### SUMMARY

It is an object of the present invention to address the problems of the state of the art cooling devices, such as cryocoolers for example. It is more particularly an object of the present invention to provide a cooling device which can be inserted into and removed from a vacuum chamber or into a boot of a vacuum chamber, and which can be easily and/or quickly repaired and/or maintained, particularly for its moving parts.

The invention is defined by the independent claims. The dependent claims define advantageous embodiments.

According to the present disclosure, there is provided a cooling device for cooling an object contained in a vacuum chamber, the cooling device being insertable into and removable out of a boot housed by the vacuum chamber, the cooling device extending along a longitudinal axis and presenting a proximal portion, an intermediate portion and a distal portion, the distal portion comprising a first cold station and a first coupler thermally connected to the first cold station. The first coupler comprises at least two mobile contacts that are thermally connected to the first cold station, and the cooling device comprises a driving means and a mechanical transmission connecting the driving means to the at least two mobile contacts, the driving means and the mechanical transmission being configured to move each of the at least two mobile contacts radially inwardly and/or outwardly.

In the context of the present invention, thermally connected means that there is a conductive thermal link between the connected parts, so that heat may be transmitted by conduction between the connected parts.

In the context of the present invention, and as is generally known in the art of machinery and control, a driving means is a component that is adapted to produce force or torque on the mobile contacts via the mechanical transmission, or displacement of the mobile contacts in a controlled or controllable way, when input power is supplied to the driving means. Input power may for example be electric power, or pneumatic power, or hydraulic power, or human power (i.e. power applied by a human being).

Since the driving means and the mechanical transmission are part of the cooling device, it becomes easy to maintain or to replace the driving means and/or (parts of) the mechanical transmission when the cooling device is removed out of the boot of the vacuum chamber. Because of the presence of the driving means, it becomes furthermore possible to apply a controlled force on the mobile contacts so as to move these mobile contacts radially inwardly and/or outwardly in a controlled way and without being dependent on any kind of heating or cooling of any part.

In some embodiments, the driving means is a manual driving means, such as a crank or a lever or a wheel for example, and which can be operated by a human being.

In some embodiments, the driving means is a motor, such as for example an electric motor, or an electromagnet, or a hydraulic motor, or a pneumatic motor, or a hydraulic cylinder, or a pneumatic cylinder.

In some embodiments, the driving means and the mechanical transmission are configured to drive and to move each of the at least two mobile contacts radially inwardly to a thermally disconnected configuration and to drive and to move each of the at least two mobile contacts radially outwardly to a thermally connected configuration, or vice-versa.

In some embodiments, the driving means is configured to drive and to move each of the at least two mobile contacts simultaneously and radially inwardly to a thermally disconnected configuration and to drive and to move each of the at least two mobile contacts simultaneously and radially outwardly to a thermally connected configuration, or vice-versa. By simultaneously it is meant that the mobile contacts are moved all together and synchronously from their respective initial starting positions, either outwardly or inwardly. This allows for a good centering of the cooling device after it has been inserted into the boot of the vacuum chamber and following to the actuation of the driving means.

In some embodiments, the driving means is arranged at the proximal portion of the cooling device and the mechanical transmission comprises a drive shaft connecting the driving means to the first coupler. This allows for an easy access to the driving means from the outside of the vacuum chamber when the cooling device is inserted into the boot of the vacuum chamber.

According to the present disclosure, there is also provided a charged particle accelerator comprising: a vacuum chamber, a main superconducting magnet arranged into the vacuum chamber, a boot housed into the vacuum chamber and presenting an opening to the ambient, one or more thermal links between the superconducting magnet and the boot or part of the boot, and a cooling device as described herein.

### BRIEF DESCRIPTION OF THE DRAWINGS

These and further aspects of the invention will be explained in greater detail by way of examples and with reference to the accompanying drawings in which:
**Fig. 1** schematically shows a general cutaway view of a cooling device according to the invention when in operational position inside a boot of a vacuum chamber;
**Fig. 2** schematically shows a general cutaway view of the cooling device of Fig. 1, when being partially retracted out of the boot;
**Fig. 3** schematically shows a partial cutaway view of a first embodiment of a cooling device according to the invention when in operational position inside a boot of a vacuum chamber;
**Fig. 4** shows a cross-sectional view of the cooling device of Fig. 3;
**Fig. 5** schematically shows a partial cutaway view of a second embodiment of a cooling device according to the invention when in operational position inside a boot of a vacuum chamber;
**Fig. 6** shows a cross-sectional view of the cooling device of Fig. 5;
**Fig. 7** schematically shows a cutaway view of a preferred embodiment of the cooling device of Fig. 3;
**Fig. 8** schematically shows a cutaway view of a preferred embodiment of the cooling device of Fig. 5;
**Fig. 9** schematically shows a cross-sectional view of another embodiment of a cooling device according to the invention.
**Fig. 10** schematically shows a partial view of a third embodiment of a cooling device according to the invention when in operational position inside a boot of a vacuum chamber;
**Fig. 11** schematically shows a cutaway view of a preferred embodiment of the cooling device of Fig. 7;
**Fig. 12** schematically shows a cross-sectional view of a fourth embodiment of a cooling device according to the invention when in operational position inside a boot of a vacuum chamber;
**Fig. 13** schematically shows a cross-sectional view of a fifth embodiment of a cooling device according to the invention when in operational position inside a boot of a vacuum chamber;
**Fig. 14** schematically shows a charged particle accelerator including an embodiment of a cooling device according to the invention;
**Fig. 15** schematically shows a charged particle accelerator including another embodiment of a cooling device according to the invention.

The drawings of the figures are neither drawn to scale nor proportioned. Generally, similar or identical components are denoted by the same reference numerals in the figures.

### DETAILED DESCRIPTION

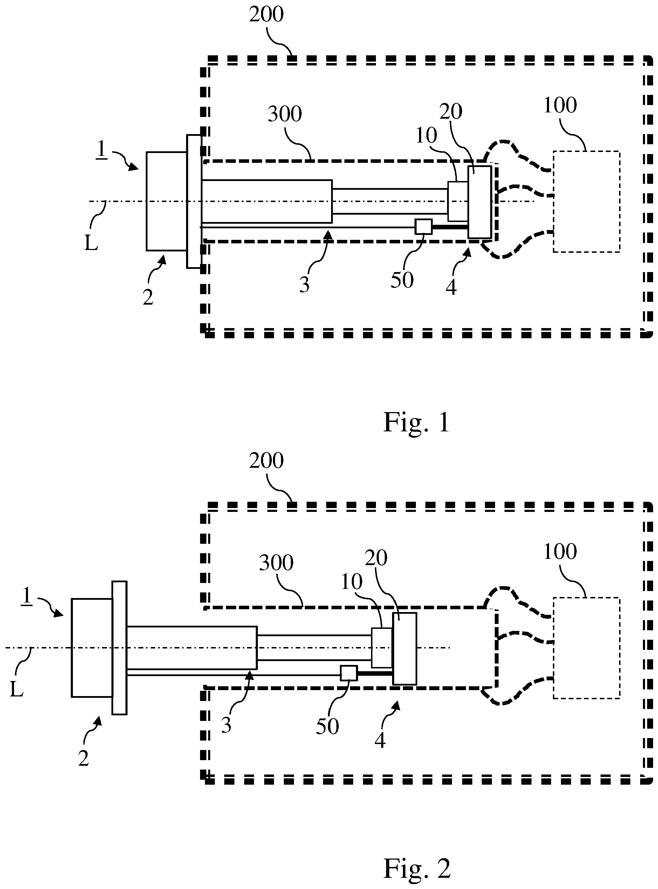

Fig.1 schematically shows a general cutaway view of a cooling device (1) according to the invention (hereafter sometimes also referred to as "the cryocooler"), when for example inserted into an operational position into a boot (300) housed into a vacuum chamber (200) (hereafter sometimes also referred to as "the cryostat"), the vacuum chamber containing the object to be cooled (100) by the cooling device. The object to be cooled inside the vacuum chamber may for example be a superconducting magnet, such as for example the main magnet of a particle accelerator, such as for example the main magnet of a cyclotron, for example of a synchrocyclotron.

Fig.2 schematically shows a general cutaway view of the cooling device (1) of Fig.1, when being partially retracted out of the said boot (300). As can be seen on this figure, the whole cooling device (1) can be removed longitudinally and without breaking the vacuum of the vacuum chamber (200).

As can be seen on Fig.1 and Fig.2 the cooling device (1) is longitudinally insertable into and removable out of the boot (300) housed by the vacuum chamber (200). On Fig.1 and Fig.2, the vacuum chamber (200) and the boot (300) are shown in dotted lines and are not part of the cooling device (1).

The cooling device (1) extends along a longitudinal axis (L) and presents a proximal portion (2), an intermediate portion (3) and a distal portion (4). The proximal portion (2) is that portion of the cooling device (1) which is located outside of the vacuum chamber (200) when the cooling device (1) is inserted into the boot (300) and it may for example comprise a flange and a joint so as to provide for an airtight connection between the cooling device (1) and the boot (300) when the cooling device (1) is inserted into the boot (300). In case of a cryocooler, the proximal portion (2) is sometimes called the head of the cryocooler. The distal portion (4) comprises a first cold station (10) and a first coupler (20) thermally connected to the first cold station (10). In operation, the cooling device (1) cools the first cold station (10), which in turn allows to cool the object to be cooled (100) into the cryostat through a thermal link (shown by three serpentine-like dotted lines) between the first cold station (10) and the object to be cooled (100), via the first coupler (20) and the boot (300) or a part thereof.

This is generally known in the art and will hence not be described further.

A number of exemplary embodiments of a cooling device (1) according to the invention will now be described in more detail.

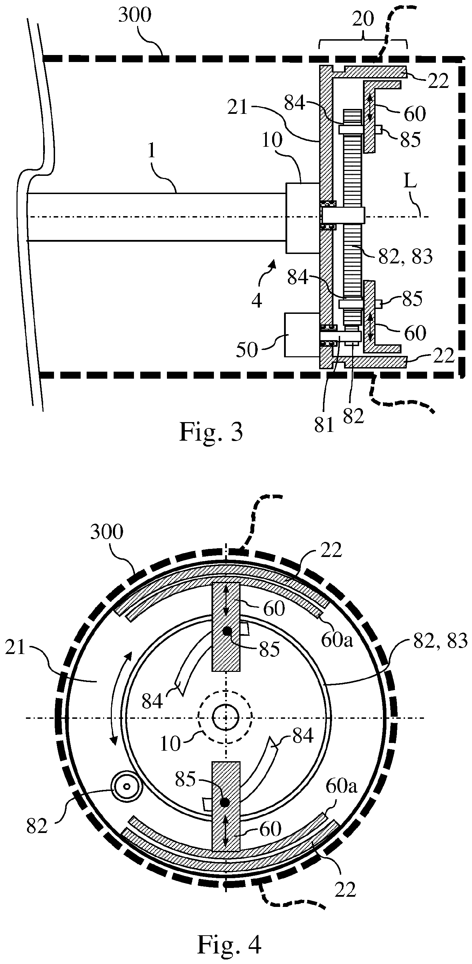

A cutaway view of a first embodiment of a cooling device (1) (or cryostat) according to the invention is shown on Fig. 3 and a cross-sectional view is shown in Fig.4. It is the same as the cooling device shown on Fig.1 and Fig.2, but with more details on the distal part of it, which comprises the first coupler (20) and its driving means (50) and the mechanical transmission ().

In this first embodiment, the first coupler (20) comprises a cup-shaped part (21) which is attached and thermally connected to the first cold station (10). The cup-shaped part (21) may alternatively form an integral part of the first cold station (10).

This cup-shaped part (21) presents at least two flexible extensions (22) extending longitudinally and arranged radially opposite to each other. In this example, the cup-shaped part (21) comprises two flexible extensions. The flexible extensions (22) are adapted to be deformed flexibly (i.e. elastically) and radially outwards or inwards (upwards and/or downwards on the cutaway views of Fig.3 and Fig.4). These flexible extensions constitute the mobile contacts (22) which can come in thermal conductive contact with the boot (300) when they are deformed, as will be explained hereafter.

The first coupler (20) also comprises at least two pushers (60) arranged in front of respectively the at least two mobile contacts (22). In the present example, the first coupler (20) comprises two pushers (60) arranged in front of respectively the two mobile contacts (22) to cooperate respectively with the two mobile contacts (22). When the two pushers (60) are moved radially outwards, as shown by the two arrows on Fig. 3 and Fig.4, they will push respectively against the two mobile contacts (22) and deform the latter until they make a thermal contact with the inner walls of the boot (300). When such thermal contact is established, heat can be conducted from the walls of the boot (300) through the two mobile contacts (22) and to the first cold station (10), allowing to cool the object to be cooled (100) into the cryostat. Since the mobile contacts (22) are arranged opposite to each other, their radial outward movement will furthermore help in centering the cooling device (1) into the boot (300) after it has been inserted into the boot (300). When the two pushers (60) are thereafter moved radially inwards, they will release the pressure against the two mobile contacts (22) so that the two mobile contacts (22) will elastically return to their initial position, or at least will loosen the contact with the inner walls of the boot (300). When this contact is loosened, it will be possible to retract the cooling device (1) from the boot (300) for repair or maintenance for example.

The cooling device (1) may also comprise guiding means (not shown) to guide each of the two pushers (60) in a radial direction only (i.e. with only one degree of freedom).

The cooling device (1) also comprises a driving means (50) and a mechanical transmission connecting the driving means (50) to the at least two mobile contacts (22), the driving means (50) and the mechanical transmission being configured to move each of the at least two mobile contacts (22) radially inwardly and/or outwardly.

In the example of Fig.3 and Fig.4, the driving means (50) comprises a motor, such as an electric motor for example, and the mechanical transmission comprises a drive shaft (81), a geartrain (82) and the two pushers (60), all arranged at the distal portion (4) of the cooling device (1). The drive shaft (81) connects the motor to the geartrain (82).
The geartrain (82) has a gear ratio of R. In some examples the value of R is greater than one, preferably greater than five, preferably greater than ten. This allows to increase the torque applied on the driven wheel (83) of the geartrain (82) and hence the force applied on the pushers (60). In this example, the drive shaft (81) is mounted on a first bearing that is mounted on a disk-shaped portion of the cup-shaped part (21), and an axle of the driven wheel (83) of the geartrain (82) is mounted on a second bearing that is mounted on a central part of the disk-shaped portion of the cup-shaped part (21). In this example, the motor is attached on the disk-shaped portion of the cup-shaped part (21) but it may also be attached on another fixed part of the cooling device (1).

The driven wheel (83) of the geartrain (82) is configured to cooperate with the two pushers (60) to move the two mobile contacts (22) of the first coupler (20) radially inwardly and/or outwardly. To obtain such cooperation and movements, the driven wheel (83) of the geartrain (82) may for example comprise two arcuate holes (84), each arcuate hole having one end at a first distance from a center of the driven wheel (83) and an opposite end at a second distance from the center of the driven wheel (83), the second distance different from the first distance, and the two pushers (60) each comprise an axial extension (85), such as a rod for example, cooperating respectively with a corresponding one of the two arcuate holes (84). Hence, when the motor is controlled to rotate in one direction, the driven wheel (83) of the geartrain (82) will force a radial outward movement of the two pushers (60), thereby pushing on the mobile contacts (22) to establish the thermal connection of the latter with the boot (300). When the motor is controlled to rotate in an opposite direction, the driven wheel (83) of the geartrain (82) will force a radial inward movement of the pushers (60), thereby loosing the connection of the mobile contacts (22) with the boot (300).

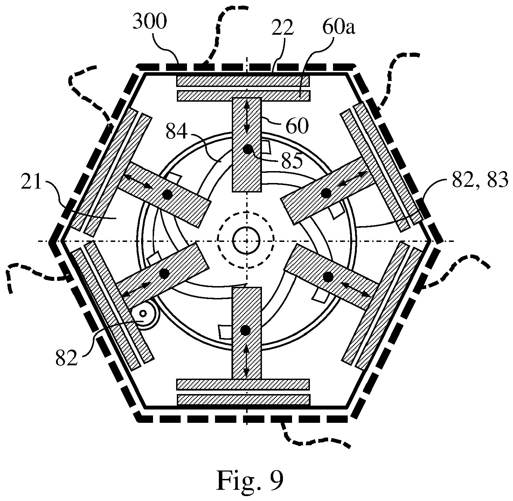

Fig. 4 shows a cross-sectional view of the cooling device of Fig. 3. On this figure, one can better see the two arcuate holes (84) of the driven wheel (83) of the geartrain (82) and how they cooperate with the pushers (60) and their axial extensions (85). In this example, the mobile contacts (22) and the distal parts (60a) of the pushers (60) all have an arcuate shape corresponding to a shape of the inner wall of the boot (300) at their contact area, in order to obtain a good thermal contact. Alternatively, in case an inner wall of the boot (300) presents flat or plane portions at the contact regions, the distal parts (60a) of the pushers (60) and the mobile contacts (22) may all have a flat shape. This may for example be the case when the inner wall of the boot (300) has a square or rectangular or polygonal cross-section at the contact regions (as seen on the example of Fig. 9 described hereafter). By "contact area" or "contact region", one has to understand the area or region where the thermal contact is made between the first coupler (20) and the boot (300) when the driving means (50) of the cooling device (1) is actuated.

In some examples, each mobile contact is mechanically connected to its corresponding pusher (60), for example through a flexible mechanical link or an articulated mechanical link between both. Hence, when the driving means (50) causes to pull on the pushers (60) to move them radially inwards, each pusher (60) will also pull on its corresponding mobile contact to move it inwards. This has the advantage that it forces the breaking of the contact between each mobile contact (22) and the boot (300), which may be useful in case this contact becomes frozen for example.

In the example of Fig.3 and Fig.4, the driving means (50) and the mechanical transmission are hence configured to drive and to move each of the at least two mobile contacts (22) radially inwardly to a thermally disconnected configuration and to drive and to move each of the at least two mobile contacts (22) radially outwardly to a thermally connected configuration.

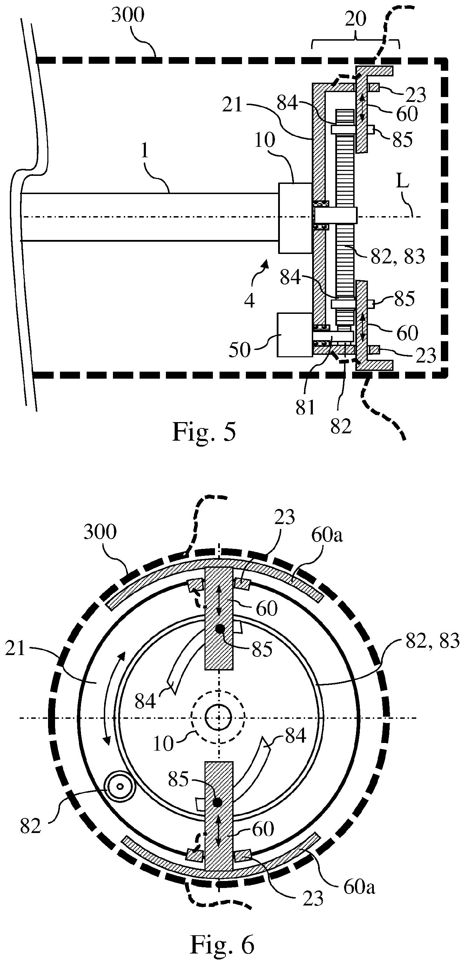

A cutaway view of a second embodiment of a cooling device (or cryostat) according to the invention is shown on Fig. 5 and Fig.6. It is the same as the cooling device (1) shown on Fig.3 and Fig.4, except that the first coupler (20) is slightly different in this second embodiment. In this second embodiment, the cup-shaped part (21) of the first coupler (20) does not present the flexible extensions arranged around the pushers as is the case with the first embodiment, but it presents longitudinal guiding extensions (23), as shown on Fig. 5 and Fig.6. Each guiding extension comprises for example a slot through which respectively each pusher (60) freely and snugly passes, so that each pusher (60) is in sliding thermal contact with each corresponding guiding extension, respectively. In other words, in this second embodiment, the pushers (60) are the mobile contacts. Alternatively, or complementary, flexible thermal links may be arranged respectively between the two guiding extensions (23) and the two pushers (60), respectively, as shown by two dotted lines on Fig. 5 and Fig. 6. Accordingly, heat can be transferred by conduction between the boot (300) and the first cold station (10) via the two mobile contacts (the two pushers (60)) when the two mobile contacts (the two pushers (60)) are brought in contact with the boot (300). The driving means (50) is here the same as the driving means (50) of the first embodiment. When powered, the driving means (50) will hence also move the pushers (60) radially outwards and/or inwards. When the driving means (50) is controlled to move the pushers (60) radially outwards, the pushers (60) will directly contact the inner wall of the boot (300), so that, when the cooling device (1) is in operation, heat will be transferred from the object to be cooled (100) to the first cold station (10), through the boot (300) (or part of it) and the first coupler (20). When the driving means (50) is controlled to move the pushers (60) radially inwards, the pushers (60) will be moved away from the inner wall of the boot (300), so that the thermal connection will be loosened and so that the cooling device (1) may be pulled out of the boot (300).

Fig. 6 shows a cross-sectional view of the cooling device of Fig. 5. On this figure, one can see another view of the guiding extensions (23). In this second embodiment, the distal parts (60a) of the mobile contacts (the pushers (60)) also have an arcuate shape corresponding to a shape of the inner wall of the boot (300) at their contact area, in order to obtain a good thermal contact. Alternatively, in case an inner wall of the boot (300) presents flat or plane portions at the contact regions, the distal parts (60a) of the mobile contacts (the pushers (60)) may all have a flat shape. This may for example be the case when inner wall of the boot (300) has a square or rectangular or polygonal cross-section at the contact regions, as shown in Fig.9 for example.

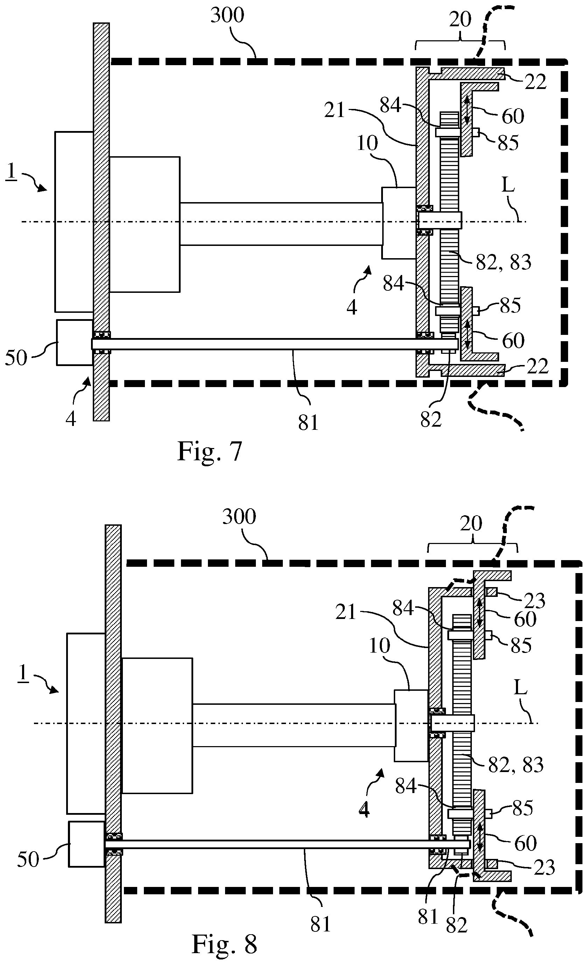

Fig. 7 schematically shows a cutaway view of a preferred embodiment of the cooling device of Figure 3. It is the same as the embodiment of Fig. 3, except that the driving means (50) is arranged at the proximal portion (2) of the cooling device (1) (namely on the left side of the cooling device as shown on Fig.7). In this example, the driving means (50) is more specifically arranged outside of that part of the cooling device which is to be inserted into the boot (300), so that the driving means (50) is accessible from the outside of the boot (300) or from outside of the vacuum chamber (200) when the cooling device (1) is inserted into the boot (300).

Fig. 8 schematically shows a cutaway view of a preferred embodiment of the cooling device of Figure 5. It is the same as the embodiment of Fig. 5, except that the driving means (50) is arranged at the proximal portion (2) of the cooling device (1) (namely on the left side of the cooling device as shown on Fig.8). In this example, the driving means (50) is more specifically arranged outside of that part of the cooling device which is to be inserted into the boot (300).

In the embodiments of Fig.7 and Fig.8, the driving means (50) may be a motor or a manual driving means (50), such as a crank or a lever or a wheel for example, and which can be operated by a human being from outside of the vacuum chamber (200) while the cooling device (1) is inserted in operational position into the boot (300) of the vacuum chamber (200).

In the embodiments described so far, there are two mobile contacts (22), but there may be more of them. In each embodiment, there may be between two and sixteen mobile contacts (22). In some examples, there are a pair number of mobile contacts (22), more preferably eight mobile contacts (22).

In each embodiment, the at least two mobile contacts (22) are angularly evenly spaced around the longitudinal axis (L) of the cooling device (1) and are arranged at equidistance from the longitudinal axis (L).

Fig. 9 schematically shows a cross-sectional view of another embodiment of a cooling device according to the invention. It is the same as the embodiment of Fig. 3 or of Fig.7, except that the coupler counts six mobile contacts (22) and six pushers (60) instead of two, and except that the mobile contacts (22) and the distal parts (60a) of the pushers (60) all have a flat or plane shape in order to make a contact with each other and with the inner surface of the boot (300) when the latter has a hexagonal cross section. As one can see on Fig.9, the mobile contacts (22) are angularly evenly spaced around the longitudinal axis (L) of the cooling device (1), in this example with an angle of 60° between each pair of adjacent mobile contacts (22). The mobile contacts (22) are also arranged at equidistance from the longitudinal axis (L) of the cooling device (1).

It will be obvious that the coupler may comprise more or less than six mobile contacts (22) and that there may be other polygonal cross sections for the inner wall of the boot (300) with corresponding arrangements of the mobile contacts (22) and of the pushers (60).

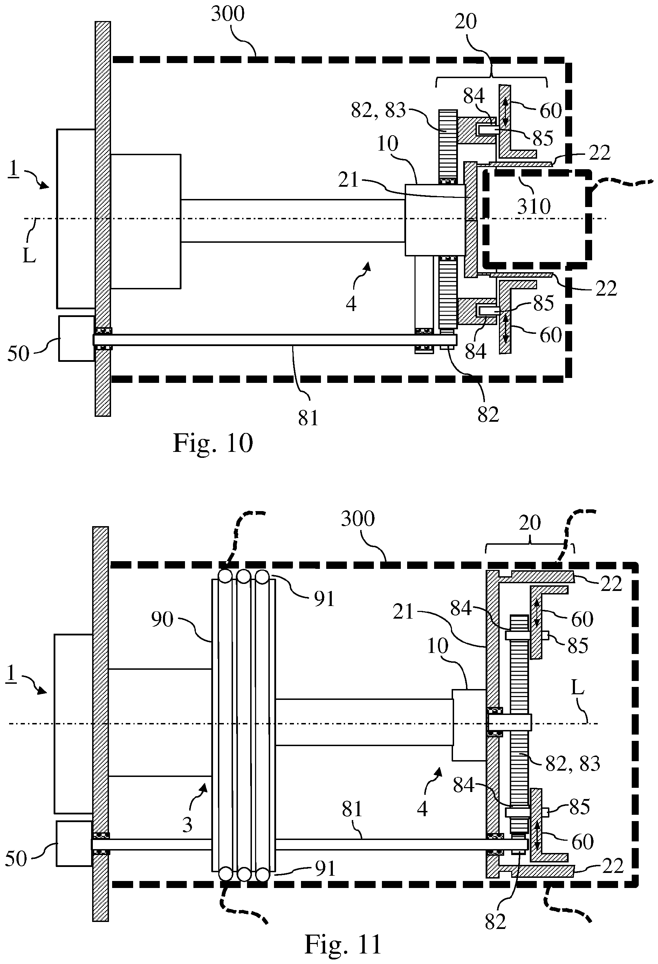

A cutaway view of a third embodiment of a cooling device according to the invention is shown on Fig. 10. It is the same as the cooling device shown on Fig.1 and Fig. 3, except for some differences on the first coupler side. In this third embodiment, the cup-shaped part (21) of the coupler is still in thermal contact with the first cold station (10) of the cooling device (1), but it is arranged more centrally and between the pushers (60) who surround the flexible extensions (the mobile contacts (22)) of the cup-shaped part (21) of the first coupler. This configuration is particularly suitable when the boot (300) presents a centrally arranged thermal port (310) at its distal end, such as a cylinder for example. The driving means (50) is substantially the same as with the other embodiments. Here, the driving means (50) is configured to drive and to move each of the at least two mobile contacts (22) radially inwardly to a thermally connected configuration and to drive and to move each of the at least two mobile contacts (22) radially outwardly to a thermally disconnected configuration.

In some examples, each mobile contact (22) is mechanically connected to its corresponding pusher (60), for example through a flexible mechanical link or an articulated mechanical link between both. Hence, when the driving means (50) causes to drive the pushers (60) to move them in one radial direction or in the opposite radial direction, the pushers (60) will also drive their corresponding mobile contacts (22) in the same directions.

In the example of Fig. 10, the driving means (50) and the mechanical transmission are hence configured to drive and to move each of the at least two mobile contacts (22) radially inwardly to a thermally connected configuration and to drive and to move each of the at least two mobile contacts (22) radially outwardly to a thermally disconnected configuration.

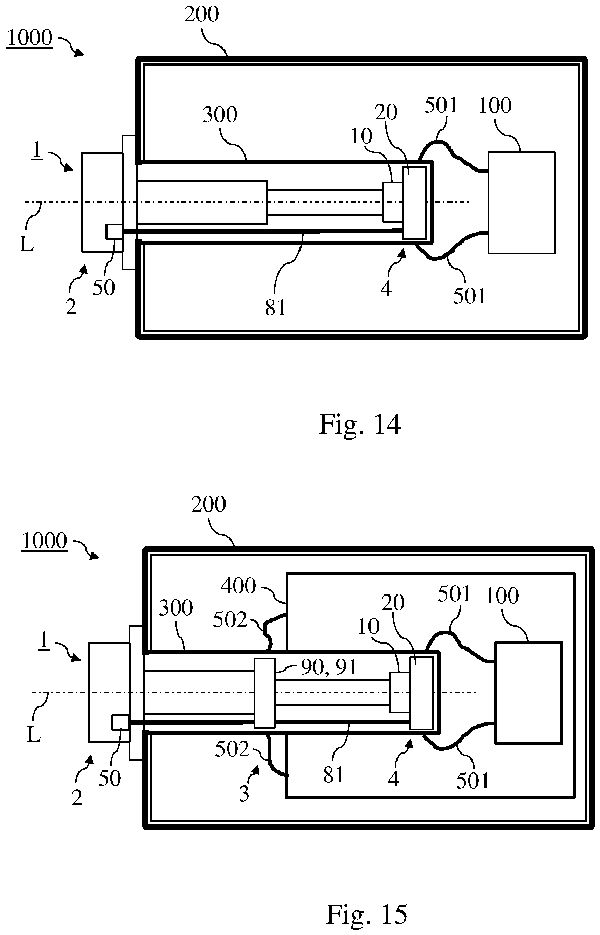

Fig. 11 schematically shows a cutaway view of a preferred embodiment of the cooling device of Fig.7. It is the same as the cooling device of Fig.7, except that the intermediate portion (3) of the cooling device (1) comprises a second cold station (90) and a second coupler (91) thermally connected to the second cold station (90). The second coupler (91) may be the same as the first coupler (20) described herein, in which case it also comes with a driving means and a mechanical transmission as described herein (though separate ones), or it may be a passive device as illustrated on Fig.1 1. In the example of Fig. 1 1, the second coupler (91) comprises a series of passive elements arranged around the second cold station (90) and in thermal contact with it. These passive elements are dimensioned and arranged such that they form a sliding contact with an inner wall of the boot (300) when the cooling device (1) is installed into the boot (300). Due to the sliding contact, heat may be transferred from the object to be cooled (100) to the second cold station (90), via the boot (300) and the passive elements. The passive elements may for example be toroidal springs, such as for example Bal Springs^{®} of the company Bal Seal. This second cold station (90) and second coupler (91) may be used to cool another object in the vacuum chamber (200), such as for example to cool a heat shield (400) included in the vacuum chamber (200) and thermally connected to a corresponding thermal conductive portion of the boot (300), as shown in relation to Fig. 15 for example.

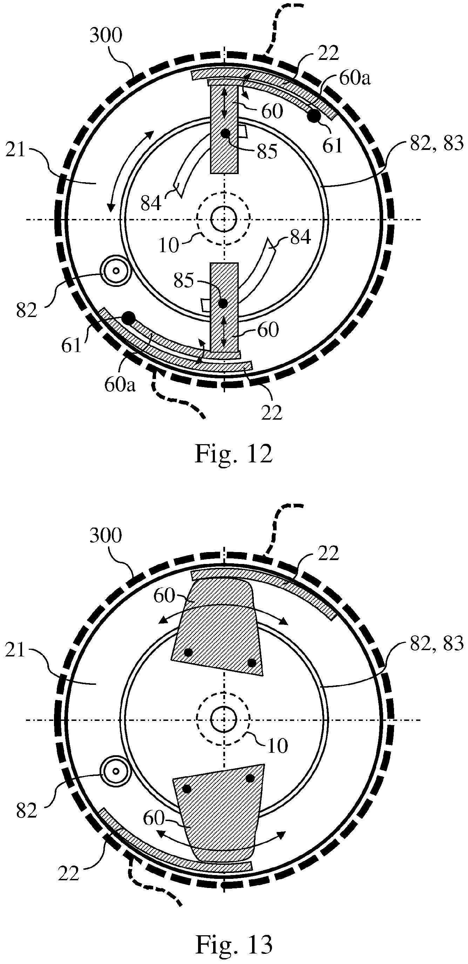

Fig. 12 schematically shows a cross-sectional view of a fourth embodiment of a cooling device according to the invention when in operational position inside a boot (300) of a vacuum chamber (200). This embodiment is the same as the other embodiments described herein, except that the pushers (60) are arranged differently and move differently when driven by the driving means (50). In this example, the distal part (60a) of a pusher (60) is rotatably mounted on the cooling device (for example on the cup-shaped part (21)), for example through a hinge (61) arranged at one extreme portion of the said distal part (60a) (the right portion on the top pusher (60) on Fig. 12), the axis of the hinge (61) being parallel to the longitudinal axis (L) of the cooling device. The other extreme portion of the said distal part (60a) which is opposite to the hinge (61) (the left portion on the top pusher (60) on Fig.12) is arranged to cooperate with one end of a radial portion of the pusher (60) (the end of the vertical portion on the top pusher (60) on Fig.12). The distal part (60a) of the pusher (60) may be a flexible part attached to the said one end of the radial portion of the pusher (60) or be flexibly linked to the said one end of the radial portion of the pusher (60). Hence, when the driving means (50) is actuated, the radial portion of the pusher (60) will push and/or pull on its distal part (60a) thereby respectively establishing or breaking the thermal contact with the boot (300) via the corresponding movable contact (22). The same holds for the other pushers (60). It will be obvious that the same arrangement can be used in the absence of the mobile contacts (22), such as is the case of the embodiments shown in Fig. 5, Fig. 6 and Fig. 8 for example.

Fig. 13 schematically shows a cross-sectional view of a fifth embodiment of a cooling device according to the invention when in operational position inside a boot (300) of a vacuum chamber (200). This embodiment is the same as the embodiments described herein in relation to Figs. 3, 4, 7 and 9 for example, except that the pushers (60) are arranged differently and move differently when driven by the driving means (50). In this example, the two pushers (60) are fixedly attached to the driven wheel (83) of the geartrain (82) and are therefore brought into rotation around the axis of the driven wheel (83) when the driving means (50) are actuated, as indicated by the double arrows of Fig. 13. The pushers (60) each have a rounded distal part which comes in contact with the corresponding mobile contacts (22) and hence push the mobile contacts (22) radially outwards when the driven wheel (83) is rotated clockwise up to the position shown in Fig.13. When the driven wheel (83) is thereafter rotated counterclockwise (or further clockwise in this case) up to an angle where the pushers (60) have no contact with the mobile contacts (22) anymore, the pressure on the mobile contacts (22) will be released, thereby loosening the thermal contact between the mobile contacts (22) and the boot (300).

Whatever the embodiment, the mechanical transmission has a transmission ratio of R, wherein R is greater than one, preferably greater than five, preferably greater than ten. As is well known, the transmission ratio R of a mechanical transmission is the ratio Wi/Wo, wherein Wi is the input speed of the mechanical transmission , and Wo is the output speed of the mechanical transmission .

In some examples, the cooling device (1) is adapted, when in operation, to cool the first cold station (10) down to a temperature between 1°K and 100°K or between 1°K and 25°K, in some examples down to a temperature between 2°K and 10°K.

In some examples, the cooling device (1) is adapted, when in operation, to cool the second cold station (90) down to a temperature between 30°K and 100°K, in some examples down to a temperature between 30°K and 60°K.

The invention also provides a charged particle accelerator (1000) comprising:
- a vacuum chamber (200);
- a main superconducting magnet (100) arranged into the vacuum chamber;
- a boot (300) housed into the vacuum chamber (200) and presenting an opening to the ambient;
- one or more thermal links between the superconducting magnet (100) and the boot (300) or part of the boot (300); and
- a cooling device as described herein and arranged into the boot (300).

As is known in the art of particle accelerators, the vacuum chamber (200) is sometimes called a cryostat, the cooling device (1) is sometimes called a cryocooler, and the main superconducting magnet is the electromagnet which imposes a trajectory of the charged particles while being gradually accelerated into the accelerator.

The thermal links are for example thermally conductive links, for example made of copper or aluminum, or liquid links, such as liquid helium for example, or a combination of those.

Fig. 14 schematically shows an exemplary charged particle accelerator (1000) including an embodiment of a cooling device (1) according to the invention. The cooling device (1) shown on Fig.14 is for example a cooling device as described in relation to Figs. 7 to 10 or Figs.12 to 13. Accordingly, heat will be transferred from the superconducting magnet (100) to the first cold station (10) of the cryocooler, via first thermal links (501), the distal portion (4) of the boot (300), and the first coupler (20), when the cryocooler is in operation. In this configuration, the cooling device is for example adapted, when in operation, to cool the first cold station (10) down to a temperature between 1°K and 100°K, or down to a temperature between 1°K and 10°K, preferably down to a temperature between 3°K and 6°K.

Fig. 15 schematically shows an exemplary charged particle accelerator (1000) including another embodiment of a cooling device according to the invention. The cooling device (1) shown on Fig.15 is for example a cooling device as described in relation to Fig. 11. In this case, the vacuum chamber (200) also houses a heat shield (400) surrounding at least the superconducting magnet. Accordingly, heat will be transferred from the superconducting magnet (100) to the first cold station (10) of the cryocooler, via the first thermal links (501), the distal portion (4) of the boot (300), and the first coupler (20), and heat will also be transferred from the heat shield to the second cold station (90) of the cryocooler, via the second thermal links (502), the intermediate portion (3) of the boot (300), and the second coupler (91), when the cryocooler is in operation. In this configuration, the cooling device (1) is for example adapted, when in operation, to cool the first cold station (10) down to a temperature between 1°K and 100°K, or down to a temperature between 1°K and 10°K, and to cool the second cold station (90) down to a temperature between 15°K and 100°K, or to a temperature between 20°K and 60°K.

In some examples, the charged particle accelerator (1000) is a cyclotron or a synchrocyclotron.

In the examples of Fig.14 and Fig.15, the superconducting magnet is thermally conductively linked to the first thermal links (501).

In other examples, the superconducting magnet may be arranged into an enclosure containing liquid Helium for example, the enclosure being housed into the vacuum chamber (200), preferably inside the heat shield (400). In such a case, the first thermal links (501) are thermally connected to a condenser arranged into the said enclosure to condense the Helium after it has evaporated due to its heating up by the superconducting magnet.

The present invention has been described in terms of specific embodiments, which are illustrative of the invention and not to be construed as limiting. Reference numerals in the claims, if any, do not limit their protective scope. Use of the verbs "to comprise", "to include", "to be composed of", or any other variant, as well as their respective conjugations, does not exclude the presence of elements other than those stated. Use of the article "a", "an" or "the" preceding an element does not exclude the presence of a plurality of such elements.
The invention may also be described as follows: a device for cooling an object (100) contained into a vacuum chamber (200), the object being a superconducting magnet for example. The cooling device (1) is insertable into and removable out of a boot (300) housed by the vacuum chamber (200), the boot or part of it being in thermal contact (conductive and/or convective) with the object to be cooled (100). A distal portion (4) of the cooling device (1) comprises a first cold station (10) and a first coupler (20) thermally connected to the first cold station (10). The first coupler (20) comprises at least two mobile contacts (22, 60) that are thermally connected to the first cold station (10), and the cooling device (1) comprises a driving means (50) and a mechanical transmission connecting the driving means (50) to the at least two mobile contacts (22, 60), the driving means (50) and the mechanical transmission being configured to move each of the at least two mobile contacts (22, 60) radially inwardly and outwardly in order to make or loosen a conductive thermal contact between the first cold station (10) and the boot (300) or part thereof.

## Claims

1. A cooling device (1) for cooling an object contained in a vacuum chamber (200), the cooling device being insertable into and removable out of a boot (300) housed by the vacuum chamber (200), the cooling device extending along a longitudinal axis (L) and presenting a proximal portion (2), an intermediate portion (3) and a distal portion (4), the distal portion (4) comprising a first cold station (10) and a first coupler (20) thermally connected to the first cold station (10),
**characterized in that** the first coupler (20) comprises at least two mobile contacts (22, 60) that are thermally connected to the first cold station (10), and **in that** the cooling device comprises a driving means (50) and a mechanical transmission connecting the driving means (50) to the at least two mobile contacts (22, 60), the driving means (50) and the mechanical transmission being configured to move each of the at least two mobile contacts (22, 60) radially inwardly and outwardly.

2. A cooling device according to claim 1, **characterized in that** the driving means (50) is a motor or a manual driving means.

3. A cooling device according to claim 1 or 2, **characterized in that** the driving means (50) and the mechanical transmission are configured to drive and to move each of the at least two mobile contacts (22) radially inwardly to a thermally disconnected configuration and to drive and to move each of the at least two mobile contacts (22) radially outwardly to a thermally connected configuration, or vice-versa.

4. A cooling device according to any of claims 1 to 3, **characterized in that** the driving means (50) is arranged at the proximal portion (2) of the cooling device (1) and **in that** the mechanical transmission comprises a drive shaft (81) connecting the driving means (50) to the first coupler (20).

5. A cooling device according to any of claims 1 to 4, **characterized in that** the mechanical transmission has a transmission ratio of R wherein R is greater than 1.

6. A cooling device according to claim 5, **characterized in that** the mechanical transmission comprises a geartrain (82) arranged at the distal portion (4) of the cooling device (1) and **in that** a driven wheel (83) of the geartrain (82) is configured to cooperate with the at least two mobile contacts (22) of the first coupler (20).

7. A cooling device according to claim 6, **characterized in that** the driven wheel (83) of the geartrain (82) comprises at least two arcuate holes (84), each arcuate hole having one end at a first distance from a center of the driven wheel (83) and an opposite end at a second distance from the center of the driven wheel (83), the second distance different from the first distance, and **in that** the mechanical transmission comprises at least two pushers (60) arranged in front of respectively the at least two mobile contacts (22), the at least two pushers (60) having each an axial extension (85) cooperating respectively with a corresponding one of the least two arcuate holes (84).

8. A cooling device according to claim 6, **characterized in that** the driven wheel (83) of the geartrain (82) comprises at least two arcuate holes (84), each arcuate hole having one end at a first distance from a center of the driven wheel (83) and an opposite end at a second distance from the center of the driven wheel (83), the second distance different from the first distance, and **in that** the at least two mobile contacts (60) each have an axial extension (85) cooperating respectively with a corresponding one of the least two arcuate holes (84).

9. A cooling device according to any of preceding claims, **characterized in that** the at least two mobile contacts (22, 60) are angularly evenly spaced around the longitudinal axis (L) of the cooling device (1).

10. A cooling device according to claim 9, **characterized in that** the at least two mobile contacts (22, 60) are between two and sixteen mobile contacts, preferably a pair number of mobile contacts, more preferably six or eight mobile contacts.

11. A cooling device according to any of preceding claims, **characterized in that** the cooling device (1) is adapted, when in operation, to cool the first cold station (10) down to a temperature between 1°K and 100°K or between 1°K and 10°K.

12. A cooling device according to any of preceding claims, **characterized in that** the intermediate portion (3) comprises a second cold station (90) and a second coupler (91) thermally connected to the second cold station (90).

13. A cooling device according to claim 12, **characterized in that** the second coupler (91) comprises a series of passive elements arranged around the second cold station (90) and in thermal contact with the second cold station (90).

14. A cooling device according to claim 13, **characterized in that** the passive elements are toroidal springs.

15. A cooling device according to any of claims 12 to 14, **characterized in that** the cooling device (1) is adapted, when in operation, to cool the second cold station (90) down to a temperature between 15°K and 100°K or between 20°K and 60°K.

16. A charged particle accelerator (1000) comprising:
- a vacuum chamber (200);
- a main superconducting magnet (100) arranged into the vacuum chamber (200);
- a boot (300) housed into the vacuum chamber (200) and presenting an opening to the ambient;
- one or more thermal links (501, 502) between the superconducting magnet (100) and the boot (300) or part of the boot (300); and
- a cooling device according to any of preceding claims and arranged into the boot (300).

17. A charged particle accelerator (1000) according to claim 16, wherein the accelerator is a cyclotron or a synchrocyclotron.
